# EUROPÄISCHE PATENTSCHRIFT

(11) **EP 1 841 385 B1**
(45) Veröffentlichungstag und Bekanntmachung des Hinweises auf die Patenterteilung: **10.03.2010**
(21) Anmeldenummer: 05707077.3
(22) Anmeldetag: 28.01.2005
(51) Int. Cl.: A61F 2/44, A61F 2/46

(54) **IMPLANTAT ZUR TRANSFORAMINALEN INTERKORPORELLEN FUSION**
IMPLANT FOR TRANSFORAMINAL INTRACORPOREAL FUSION
IMPLANT POUR L'ARTHRODESE TRANSFORAMINALE INTERCORPOREALE

(43) Veröffentlichungstag der Anmeldung: 10.10.2007
(73) Patentinhaber: Advanced Medical Technologies AG, 66620 Nonnweiler-Braunshausen (DE)
(72) Erfinder: WEILAND, Peter, 66620 Nonnweiler-Braunshausen (DE)
(74) Vertreter: Kruspig, Volkmar
(86) Internationale Anmeldenummer: PCT/EP2005/000881
(87) Internationale Veröffentlichungsnummer: WO 2006/079356

(56) Entgegenhaltungen:
- EP-A- 1 290 985
- WO-A-01/95838
- WO-A-02/17823
- WO-A-2005/041825
- FR-A- 2 841 125
- US-A1- 2004 153 065

## Beschreibung

Die Erfindung betrifft ein Implantat zur transforaminalen interkorporellen Fusion lumbaler Wirbelsäulensegmente, wobei die mit der Wirbelsäule unmittelbar in Kontakt kommenden Oberflächenbereiche zumindest abschnittsweise eine Dislokalisationssicherung aufweisen, weiterhin im oder am Implantat ein Ansatzteil für ein Setzinstrument vorgesehen ist sowie im Implantat Öffnungen oder Hohlräume zur Befüllung angeordnet sind, gemäß Oberbegriff des Patentanspruchs 1.

Bei Wirbelsäuleneingriffen wird versucht, minimalinvasiv zu arbeiten, wobei in diesem Sinne sogenannte PLIF-Operationstechniken (Posterior-Lateral-Interbody-Fusion) entwickelt wurden. Bei einer solcher Operationstechnik wird durch einen hinteren Zugang die Bandscheibe entfernt und der intervertebrale Raum mit autologem Knochen gefüllt. Diese Technik geht zurück auf Cloward, der eine solche Operation erstmals 1943 durchführte. Weiterentwicklungen der PLIF-Technik führten dazu, dass ein transforaminaler Zugang angewendet wurde. Bei dieser Technik werden mit Eigenspongiosa gefüllte Titankörbchen, sogenannte Cage, transforaminal von dorsal her eingeführt. Gleichzeitig wird eine dorsale Instrumentierung und Stabilisierung angewendet. Der Vorteil der kurz beschriebenen Methode besteht darin, dass kein transabdominaler oder retroperitonealer zusätzlicher Zugang verwendet werden muss.

Bei dem "Biotit-Cage" der Firma Ulrich dient dieser als Bandscheibenersatz für die lumbale Wirbelsäule. Der Cage wird über einen dorsalen Zugang eingesetzt, wobei die Anwendung paarweise oder einzeln möglich ist. Dieser vorbekannte Cage hat eine im wesentlichen U-Form und weist eine Dislokalisationsicherung durch Riefenstruktur auf. Das Implantatmaterial ist beim vorgenannten Cage Titan bzw. eine Titanlegierung, so dass relativ große radiologische Fenster zur Kontrolle der Fusion erforderlich werden.

Weiterhin bekannt ist ein von der Firma Stryker Orthopaedic, USA, vertrieber Cage in Form eines kubischen Implantats, das paarweise sowohl von anterior als auch von posterior in den Zwischenwirbelraum implantiert werden kann.

Bezüglich des Standes der Technik sei noch auf DE 43 28 062 A1 aufmerksam gemacht, die ein Implantat zum Ersatz von Wirbelkörpern und/oder zur Stabilisierung und Fixierung der Wirbelsäule betrifft. Bei diesem Implantat ist auf einen Stützstab quer zur Stabachse ein Implantatkörper aufgeschoben. Der Implantatkörper gemäß DE 43 28 062 A1 ist mit einer Oberflächenstruktur an seinem zur Anlage an den an das Implantat angrenzenden Wirbelkörpern versehen, so dass sich eine gegenseitige Fixierung der aneinanderliegenden Flächen des Implantatkörpers einerseits und der Wirbelkörper andererseits einstellt.

Zum Stand der Technik gehören ebenfalls höhenverstellbare Wirbelkörper-Implantate für den Ersatz eines oder mehrerer Wirbelkörper, beispielsweise gemäß DE 44 23 257 A1, DE 195 19 101 A1 oder aber DE 195 09 317 A1. Die Fertigung derartiger höhenvariabler Implantate ist jedoch sehr kostenintensiv und in ihrer Handhabung kompliziert.

Beim deutschen Gebrauchsmuster DE 296 16 778 U1 ist ein Wirbelkörper-Platzhalter offenbart, der z.B. nach einer Wirbelkörperresektion eingesetzt wird und der anstelle des fehlenden Wirbelkörpers tritt. Derartige Platzhalter sollen aus einem körperverträglichen Material bestehen, wobei die Stirnenden eine unregelmäßige Kante bilden und die Wandung des Platzhalters Löcher aufweist, so dass genügend Raum zur Aufnahme von Knochensubstanz geschaffen ist.

Zum Einsetzen des Platzhalters nach DE 296 16 778 U1 wird ein Werkzeug verwendet, das einen relativ langen Schaft aufweist, wobei der Schaft am freien Ende einen Gewindezapfen besitzt. Der Gewindezapfen kann mit einem Gewindeloch des hülsenförmigen Körpers in Eingriff gebracht werden, um diesen in der vorgegebenen Lage zwischen zwei benachbarten Wirbelkörpern in den Raum des resistierten Wirbelkörpers einzusetzen.

Aus der US 2004/0153065 A1 ist ein Implantat mit Setzvorrichtung bekannt, welches eine annähernde Sichelform besitzt und wobei an einem Implantatende ein Ansatz vorhanden ist. Der Ansatz weist Durchgangsbohrungen auf, die in der Lage sind, ein entsprechend geformtes Endstück des Setzinstruments aufzunehmen. Hierdurch besteht die Möglichkeit, beim operativen Einsetzen des Implantats unter Nutzung des Setzinstruments Verschwenkbewegungen auszuführen.

Bei dem Implantat zur interkorporellen Fusion von Wirbelsäulensegmenten nach WO 02/17823 A1 besitzen die mit der Wirbelsäule in Kontakt kommenden Oberflächenbereiche zackenartige Dislokalisationssicherungen. Ein Ende des Implantats weist eine Stirnfläche auf, die mit einer Innengewindebohrung versehen ist, um ein entsprechendes Gewindeende eines Winkelstücks des zugehörigen Setzinstruments aufzunehmen.

Die Lehre nach der EP 1 290 985 A2 offenbart ein Wirbelkörperimplantat, dessen Schenkel durch ein zwischen den Schenkeln beweglich und verschiebbar gehaltenes Zwischenstück gespreizt werden können, wofür ein spezielles Setzinstrument zum Einsatz kommt.

Die nachveröffentlichte WO 2005/041825 zeigt ein Implantat zur transforaminalen interkorporellen Fusion lumbaler Wirbelsäulensegmente, wobei das Implantat eine Sichelform besitzt. Bei dem dortigen Implantat sind beide Enden mit einem Langloch versehen, um wahlweise ein Achsenende eines Setzinstruments aufzunehmen. Aufgrund der beidseitig vorgesehenen Ausführung des Implantats zur Aufnahme des Setzinstruments ist es nicht möglich, dem Einführende des Implantats eine solche Gestalt zu geben, die ein Einsetzen des Implantats ohne Spreizen der Wirbelsäulensegmente ermöglicht.

Aus dem Vorgenannten ist es Aufgabe der Erfindung, ein weiterentwickeltes Implantat zur transforaminalen interkorporellen Fusion lumbaler Wirbelsäulensegmente anzugeben, wobei das Implantat eine hohe Primärstabilität aufweisen soll und in der Anwendung ein einfaches operatives Vorgehen möglich wird.

Die Lösung der Aufgabe der Erfindung erfolgt mit einem Implantat gemäß Merkmalskombination nach Patentanspruch 1, wobei die Unteransprüche zweckmäßige Ausgestaltungen und Weiterbildungen umfassen.

Ausgehend von einem Implantat, welches bei den mit der Wirbelsäule unmittelbar in Kontakt kommenden Oberflächenbereichen zumindest abschnittsweise eine Dislokalisationssicherung aufweist und das ein Ansatzteil für ein Setzinstrument besitzt, weist der weiterentwickelte Implantatkörper eine Sichelform auf, wobei die Sichelwölbung ventral und die Sichelinnenseite dorsal orientiert ist.

Das spezielle Ansatzteil ist an einem Sichelende, als Drehgelenk wirkend, befindlich, wobei das diesem gegenüberliegende Sichelende eine schnabelartige, spitz zulaufende, als Einführhilfe dienende Gestalt aufweist. Zwischen den Sichelwänden des Implantatkörpers ist mindestens eine Befüllöffnung zur großvolumigen Aufnahme von Knochensubstanz vorhanden.

Das Ansatzteil ist als in eine Durchgangsbohrung aufgenommener dreh- oder verschwenkbarer Bolzen ausgeführt, wobei der Bolzen eine senkrecht zur Längsachse verlaufende Einstecköffnung für ein Instrument besitzt.

Am Ansatzteil-Sichelende ist eine Aussparung als Zugang zum Bolzen und dort vorhandener Einstecköffnung für das vorerwähnte Setzinstrument vorgesehen.

Bevorzugt besteht das Implantat aus einem bioelastischen Kunststoff, insbesondere Polyetheretherketon (PEEK), jedoch sind auch andere geeignete Implantätmaterialien denkbar.

In dem bioelastischen Kunststoff sind Röntgenmarker eingebracht. Diese Röntgenmarker können im ventralen medialen Teil in vertikaler Ausrichtung und an der Implantatspitze in horizontaler sagittaler Ausrichtung befindlich sein.

Auch besteht erfindungsgemäß die Möglichkeit, den Bolzen als Drehteil aus einem Röntgen-nachweisbaren Material auszuführen.

Die Strukturen zur Dislokalisationssicherung sind bevorzugt in Pyramiden- oder Kegelstumpfform oder in Form sphärischer, geschnittener Körper realisiert.

Die Einstecköffnung des Bolzens weist ein Innengewinde auf.

Das Setzinstrument für das Handling des vorstehend beschriebenen Implantats besteht aus einem Schaft und einer Hülse, wobei die Hülse einen Stift mit Gewindeende aufnimmt und das Gewindeende zum Innengewinde der Einstecköffnung im Bolzen komplementär ausgebildet ist.

Weiterhin kann durch eine Drehbewegung der Stift mit Gewindeende längsverschieblich zur Hülse bewegt werden. Am Vorderende der Hülse ist eine Anschlagfläche für das Implantat angeordnet, so dass das über den Stift gehaltene Implantat in der jeweiligen Winkellage über den Bolzen und die Anschlagfläche durch Verspannung fixierbar ist.

Es hat sich in überraschender Weise gezeigt, dass durch die Verwendung des bioelastischen Kunststoffs PEEK ideale Voraussetzungen für eine dauerhafte Fusion bestehen. Die dem Knochen ähnlichen Elastizitätseigenschaften wirken dem Einsinkend es Implantats entgegen und fördern die Fusionsneigung. Gleichzeitig sichert die Röntgentransparenz eine optimale postoperative Diagnostik, und zwar ohne dass bezüglich des Standes der Technik im Implantatmaterial größere radiologische Fenster vorhanden sein müssen.

Geeignete, auch relativ kleine und entsprechend platzierte Röntgenmarker aus z. B. Titan oder Titanlegierungsmaterial ermöglichen jederzeit die Lokalisation des Implantats.

Bezüglich der Anwendung und des Einsatzes des Implantats sei auf nachstehende Ausführungen verwiesen.

Zunächst wird das Bandscheibenfach über einen transforaminalen Zugang ausgeräumt. Hierbei kommen an sich bekannte geeignete Fasszangen bzw. geformte Küretten zum Einsatz.

Nach erfolgter Diskektomie wird das Bandscheibenfach mittels spezieller Distraktoren auf die gewünschte Höhe distrahiert. Eine geeignete Distraktionshöhe ist dann erreicht, wenn der Distraktor unter Spannung steht und ein diesbezüglich stabiles Gefühl gegeben ist. Nach erfolgter Distraktion wird mit Hilfe spezieller Probe-Implantate die notwendige Implantatgröße, insbesondere Implantathöhe verifiziert. Zum Halten dieser Probe-Implantate kann das erfindungsgemäße Setzinstrument bereits verwendet werden.

Vor der Implantation des dann größenmäßig ermittelten Implantats wird bevorzugt das Bandscheibenfach ventral und auf der gegenüberliegenden lateralen Seite des Implantats geeignetes Material, z. B. Spongiosa aufgefüllt. Der Bereich dorsal des Implantats wird ebenfalls gefüllt.

Für das Implantat, das auch gefüllt werden soll, findet eine Füllhilfe Anwendung, in die das Implantat eingelegt wird. Die Füllhilfe nimmt hierbei die Außenkontur des Implantats auf und bildet einen Halterahmen.

Nachdem mit Hilfe des Setzinstruments und der Arretierungsmöglichkeit ein geeigneter Winkel zwischen dem Instrument und dem Implantat gefunden wurde und dieser einer entsprechenden Fixierung unterworfen wird, erfolgt das Einsetzen des Implantats in das Bandscheibenfach. Durch die Implantationskurve ist es empfehlenswert, während der eigentlichen. Implantation den Winkel durch kurzzeitiges Lockern unter Nutzung der vorteilhaften Wirkungen des Setzinstruments anzupassen.

Eine endgültige Positionierung kann dann unter Zuhilfenahme geeigneter gerader oder gebogener Stößel erfolgen. Für eine korrekte Implantation zeigt die gewölbte Implantatseite nach ventral und die beiden Implantatenden nach dorsal. Das Implantat soll zum Erreichen aller angestrebten vorteilhaften Wirkungen möglichst ventral positioniert werden.
Nach der Implantation wird das verbleibende Bandscheibenfach aufgefüllt, um eine sichere Fusion zu gewährleisten.

Das Prinzip des erfindungsgemäßen Implantats beruht auf der Spannung des Annulus und der Längsbänder bei ausreichender Knochenqualität. In bestimmten Fällen sollte eine zusätzliche Stabilisierung mit einem dorsalen Fixateur erfolgen.

Wie bereits erwähnt, kann das Implantat aus einem Kunststoffmaterial, insbesondere PEEK gefertigt werden. Dieses Material ist röntgendurchlässig und kommt in den mechanischen Eigenschaften denen des Körpers sehr nahe. Zur Erleichterung der postoperativen Diagnostik können im Implantat Röntgenmarker eingebracht werden. Ein Marker befindet sich beispielsweise im ventralen medialen Teil des Implantats in vertikaler Ausrichtung und ein weiterer Marker an der Spitze des Implantats in horizontaler sagittaler Ausrichtung. Zusätzlich kann das Drehteil zur Instrumentenaufnahme als Röntgenmarker fungieren.

Die Erfindung soll nachstehend anhand eines Ausführungsbeispiels sowie unter Zuhilfenahme von Figuren näher erläutert werden.

Hierbei zeigen:

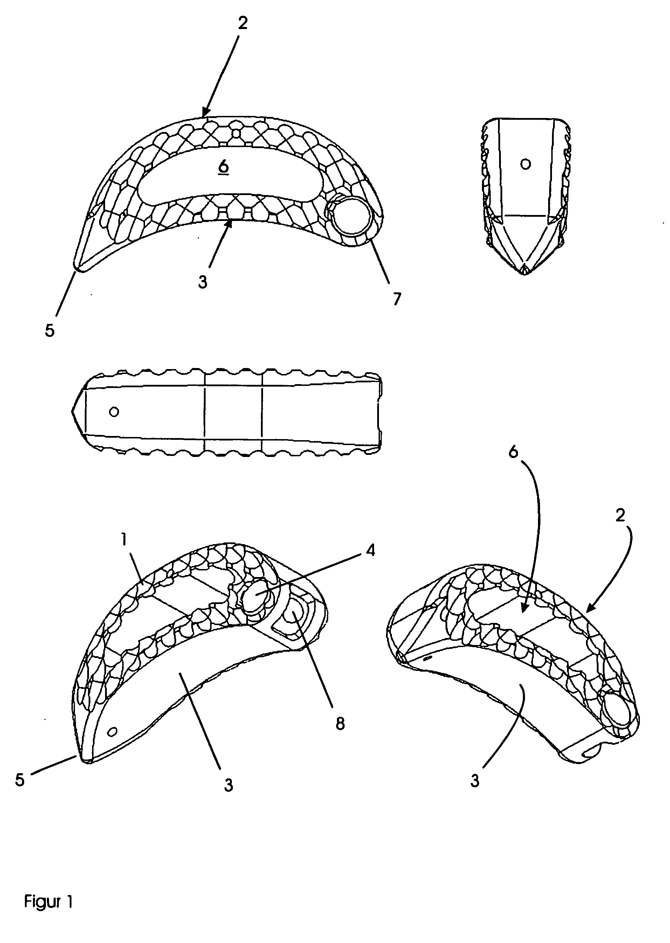

- Fig. 1: verschiedene Darstellungen des erfindungsgemäßen Implantats in Sichelform und
- Fig. 2: Seitenansicht und perspektivische Darstellung des Implantat-Setzinstruments.

Bei dem Implantat zur transforaminalen interkorporellen Fusion lumbaler Wirbelsäulensegmente gemäß Ausführungsbeispiel besitzen die mit der Wirbelsäule unmittelbar in Kontakt kommenden Oberflächenbereiche spezielle Strukturen 1 zur Dislokalisationssicherung. Diese Strukturen 1 können z. B. als Pyramidenstümpfe, Kegelstümpfe oder geschnittene sphärische Körper ausgebildet sein.

Der Implantatkörper selbst weist eine Sichelform auf, wobei die Sichelwölbung 2 ventral und die Sichelinnenseite 3 dorsal im Anwendungsfall orientiert ist.

An einem Sichelende befindet sich ein Ansatzteil 4, wobei das diesem Ansatzteil gegenüberliegende andere Sichelende eine spitz zulaufende schnabelartige Gestalt 5 besitzt.

Zwischen den Sichelwänden ist mindestens eine Befüllöffnung 6 vorgesehen.

Das Ansatzteil 4 ist als verschwenkbarer bzw. verdrehbarer Bolzen ausgeführt, der sich in einer entsprechenden Ausnehmung 7 im Implantat befindet.

Im wesentlichen senkrecht zur Längsachse des Bolzens verlaufend besitzt dieser eine Einstecköffnung 8 mit einem Innengewinde (nicht gezeigt).

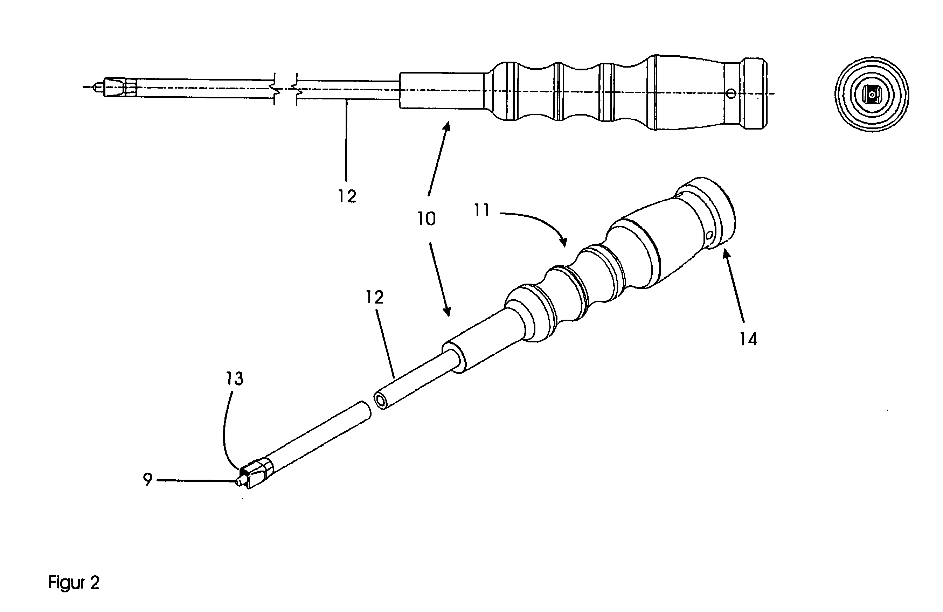

Die Einstecköffnung 8 dient der Aufnahme eines entsprechenden Endes 9 des Setzinstruments 10 (Fig. 2).

Im bevorzugt aus einem Kunststoffmaterial gefertigten Implantatkörper können verschiedene, in den Figuren nicht dargestellte Röntgenmarker eingebracht sein, so dass jederzeit eine Lokalisation des Implantats postoperativ erfolgen kann.

Das Setzinstrument 10 gemäß Fig. 2 besteht aus einem Griffteil 11 mit Hülse 12.

In der Hülse 12 ist ein Stift mit Gewindeende (Teil 9) geführt, wobei das Gewindeende zum Innengewinde der Einstecköffnung 8 im Bolzen komplementär ausgebildet ist.

Durch Drehbewegung an der Kappe 14 kann der Stift 9 mit Gewindeende bewegt werden.

Am Vorderende der Hülse 12 ist eine Anschlagfläche 13 für das Implantat angeordnet, so dass das über den Stift 9 gehaltene Implantat in der jeweiligen Winkellage über den Bolzen und die Anschlagfläche 13 fixierbar ist.

## Patentansprüche

1. Implantat zur transforaminalen interkorporellen Fusion lumbaler Wirbelsäulensegmente, wobei die mit der Wirbelsäule unmittelbar in Kontakt kommenden Oberflächenbereiche zumindest abschnittsweise eine Dislokalisationssicherung (1) aufweisen, im oder am Implantat ein Ansatzteil (4) für ein Setzinstrument (10) vorgesehen ist sowie im Implantat Öffnungen (6) oder Hohlräume zur Befüllung angeordnet sind, weiterhin der Implantatkörper eine Sichelform besitzt, wobei die Sichelwölbung (2) ventral und die Sichelinnenseite (3) dorsal orientiert ist,
das Ansatzteil (4) an einem Sichelende befindlich ist, wobei das diesem gegenüberliegende Sichelende eine schnabelartige, spitz zulaufende Gestalt (5) aufweist und
mindestens eine Befüllöffnung (6) zwischen den Sichelwänden vorgesehen ist,
**dadurch gekennzeichnet, dass**
das Ansatzteil (4) als in eine Durchgangsbohrung (7) aufgenommener dreh- oder verschwenkbarer Bolzen ausgeführt ist, wobei der Bolzen eine senkrecht zur Längsachse verlaufende Einstecköffnung (8) aufweist und am Ansatzteil-Sichelende eine Aussparung als Zugang zum Bolzen und dort vorhandener Einstecköffnung (8) für das Setzinstrument (10) vorgesehen ist, wobei die Einstecköffnung (8) im Bolzen ein Innengewinde aufweist.

2. Implantat nach Anspruch 1,
**dadurch gekennzeichnet, dass**
dieses aus bioelastischem Kunststoff, insbesondere Polyetheretherketon (PEEK) besteht.

3. Implantat nach Anspruch 2,
**dadurch gekennzeichnet, dass**
dieses Röntgenmarker aufweist.

4. Implantat nach einem der vorangegangenen Ansprüche,
**dadurch gekennzeichnet, dass**
die Strukturen zur Dislokalisationssicherung (1) eine Pyramiden- oder Kegelstumpfform aufweisen.

5. Implantat nach Anspruch 3,
**dadurch gekennzeichnet, dass**
mindestens ein Röntgenmarker im ventralen medialen Teil in vertikaler Ausrichtung und mindestens ein weiterer Marker an der Implantatspitze in horizontaler sagittaler Ausrichtung befindlich ist.

6. Implantat nach einem der vorangegangenen Ansprüche,
**dadurch gekennzeichnet, dass**
der Bolzen als Drehteil aus einem Röntgen-nachweisbaren Material besteht.

## Claims

1. Implant for the transforaminal interbody fusion of lumbar vertebral column segments, wherein at least some sections of the surface areas coming into direct contact with the vertebral column are provided with a dislocation protection (1), wherein an engagement part (4) for a positioning instrument (10) is provided in or on the implant, and holes (6) or hollow spaces are disposed in the implant for filling purposes, wherein further the implant body has the shape of a sickle, with the convexity (2) of the sickle being oriented ventrally and the inner side (3) of the sickle being oriented dorsally,
the engagement part (4) is located on one end of the sickle, and the end of the sickle opposite to the same has a beak-like, tapering shape (5), and
at least one filling hole (6) is provided between the sickle walls,
**characterized in that**
the engagement part (4) is formed as a rotatable or pivotable bolt received in a through bore (7), the bolt having an insertion opening (8) which extends perpendicular to the longitudinal axis, and the sickle end with the engagement part is provided with a recess serving as access to the bolt and to the insertion opening (8) for the positioning instrument (10), wherein the insertion opening (8) in the bolt has an inner thread.

2. Implant according to claim 1,
**characterized in that**
the implant is made of a bioelastic synthetic material, specifically polyetheretherketone (PEEK).

3. Implant according to claim 2,
**characterized in that**
the implant is provided with X-ray markers.

4. Implant according to one of the preceding claims,
**characterized in that**
the structures of the dislocation protection (1) have the shape of a truncated pyramid or truncated cone.

5. Implant according to claim 3,
**characterized in that**
at least one X-ray marker is positioned in the ventral, medial part in vertical orientation and at least one other marker at the tip of the implant in horizontal, sagittal orientation.

6. Implant according to one of the preceding claims,
**characterized in that**
the bolt is a rotary part made of an X-ray detectable material.

## Revendications

1. Implant pour la fusion intercorporelle transforaminale de segments de colonne vertébrale lombaires, dans lequel les régions de surface qui viennent en contact directement avec la colonne vertébrale présentent au moins par tronçons un moyen pour empêcher la délocalisation (1), en ce qu'il est prévu dans ou sur l'implant une pièce d'application (4) pour un instrument de pose (10), et dans lequel des ouvertures (6) ou des cavités pour le remplissage sont ménagées dans l'implant, et le corps d'implant possède une forme en croissant, la courbure du croissant (2) étant orientée de manière centrale et la face intérieure du croissant (3) étant orientée de manière dorsale,
la pièce d'application (4) se trouve à une extrémité du croissant, l'extrémité du croissant opposée à celle-ci ayant une configuration (5) en forme de bec qui converge de manière pointue, et
il est prévu au moins une ouverture de remplissage (6) entre les parois du croissant,
**caractérisé en ce que**
la pièce d'application (4) est réalisée sous la forme d'un goujon capable de tourner ou de pivoter, reçu dans un perçage traversant (7), ledit goujon présentant une ouverture d'enfichage (8) qui s'étend perpendiculairement à l'axe longitudinal, et sur l'extrémité du croissant correspondant à la pièce d'application il est prévu un évidement à titre d'accès vers le goujon et vers l'ouverture d'enfichage (8) prévue à cet endroit pour l'instrument de pose (10), et l'ouverture d'enfichage (8) dans le goujon présente un taraudage.

2. Implant selon la revendication 1,
**caractérisé en ce que** celui-ci est en matière plastique bioélastique, en particulier en polyétheréthercétone (PEEK).

3. Implant selon la revendication 2,
**caractérisé en ce que** celui-ci comporte des marqueurs pour rayons X.

4. Implant selon l'une des revendications précédentes,
**caractérisé en ce que** les structures pour empêcher la délocalisation (1) présentent une forme en pyramide ou en tronc de pyramide.

5. Implant selon la revendication 3,
**caractérisé en ce qu'**au moins un marqueur pour rayons X se trouve dans la partie médiane ventrale en orientation verticale, et au moins un autre marqueur se trouve à la pointe de l'implant en orientation horizontale sagittale.

6. Implant selon l'une des revendications précédentes,
**caractérisé en ce que** le goujon, à titre de partie rotative, est en un matériau détectable aux rayons X.
